# EUROPÄISCHE PATENTSCHRIFT

(11) **EP 2 164 468 B1**
(45) Veröffentlichungstag und Bekanntmachung des Hinweises auf die Patenterteilung: **11.01.2017**
(21) Anmeldenummer: 08801440.2
(22) Anmeldetag: 27.05.2008
(51) Int. Cl.: A61K 9/16

(54) **VERFAHREN ZUR HERSTELLUNG EINER ARZNEIMITTELDARREICHUNGSFORM**
METHOD FOR THE PRODUCTION OF A FORM OF ADMINISTRATION OF A MEDICAMENT
PROCÉDÉ DE PRODUCTION D'UNE FORME GALÉNIQUE DE MÉDICAMENT

(30) Priorität: 01.06.2007 DE 102007025858
(43) Veröffentlichungstag der Anmeldung: 24.03.2010
(73) Patentinhaber: Grünenthal GmbH, 52078 Aachen (DE)
(72) Erfinder: ARKENAU-MARIC, Elisabeth, 50931 Köln (DE); BARTHOLOMÄUS, Johannes, 52080 Aachen (DE); SCHATEIKIS, Dieter, 52223 Stolberg (DE); USTORF, Kai-Uwe, 52372 Kreuzau (DE)
(74) Vertreter: Wolff, Felix
(86) Internationale Anmeldenummer: PCT/EP2008/004193
(87) Internationale Veröffentlichungsnummer: WO 2008/145334

(56) Entgegenhaltungen:
- EP-A- 0 021 129
- EP-A- 1 449 531
- WO-A-02/35991
- WO-A-93/07859

## Beschreibung

Die vorliegende Erfindung betrifft ein Verfahren zur Herstellung einer Arzneimitteldarreichungsform die aus mindestens einem Teil besteht, dass jeweils mindestens ein Arzneimittel und mindestens einen Zusatzstoff aufweist, wobei das Arzneimittel und der Zusatzstoff gemischt und aus einer Düse als Strang extrudiert werden.

Derartige Herstellungsverfahren für Arzneimittel sind aus dem Stand der Technik beispielsweise der DE 69 509 671 T3 bekannt. In diesem Patent ist ein Verfahren beschrieben bei dem Arzneimittel und Zusatzstoffe in einem Extruder gemischt und geknetet werden und das resultierende Produkt dann aus einer Extruderdüse als Strang extrudiert wird. Dieser Strang wird in Einzelteile zerteilt. Die weitere Verarbeitung dieser Teile zu einer fertigen Arzneimitteldarreichungsform ist jedoch vergleichsweise aufwendig. Außerdem ist es gemäß dem Stand der Technik bekannt Dosierungen fester Darreichungsformen wie Tabletten oder Kapseln durch Wiegen herzustellen.

Weiterhin ist aus WO 93 7 07859 A ein Verfahren bekannt, bei dem ein Arzneimittel mit einem Zusatzstoff gemischt und aus einer Düse als Strang extrudiert wird. Der extrudierte Strang wird wiederum in gewichtsgleiche Teilchen unterteilt.

Außerdem beschreibt die Druckschrift EP 1 449 531 A ein Verfahren zur Herstellung nachhaltig freisetzender, oraler Opioidanalgetikum-Formulierungen unter Verwendung von Schmelzextrusionstechniken, um bioverfügbare Einheitsdosisprodukte bereitzustellen, die Schmerzlinderung bei einem Patienten über beispielsweise 8 bis 24 Stunden gewährleisten.

Es war deshalb die Aufgabe der vorliegenden Erfindung ein Verfahren zur Herstellung einer Arzneimitteldarreichungsform zur Verfügung zu stellen, das die Nachteile des Standes der Technik nicht aufweist.

Gelöst wird die Aufgabe mit einem Verfahren gemäß Anspruch 1.

Es war für den Fachmann überaus erstaunlich und nicht zu erwarten, dass das Verfahren sich insbesondere zur Herstellung von missbrauchsgeschützten Arzneimittelformen eignet. Mit dem erfindungsgemäßen Verfahren ist es möglich Arzneimitteldarreichungsform in einer sehr effizienten Art und Weise durchzuführen. Dadurch, dass die Teilchen gewichtsgenau abgelängt werden, kann aus ihnen erfindungsgemäß unmittelbar eine Arzneimitteldarreichungsform beispielsweise eine Tablette gepresst oder eine andere Arzneimitteldarreichungsform hergestellt werden. Ein weiterer Verfahrensschritt zur Einhaltung des Sollgewichts entfällt erfindungsgemäß.

Das erfindungsgemäße Verfahren eignet sich zur Herstellung einer Arzneimitteldarreichungsform wie beispielsweise einer Tablette oder einer multipartikulären Darreichungsform, die Granulate oder Pellets aufweist, sowie eines Zäpfchens. Des Weiteren können mit dem erfindungsgemäßen Verfahren kleine Zuschnitte hergestellt werden, die dann beispielsweise in Kapseln eingefüllt werden.

Erfindungsgemäß besteht diese Arzneimitteldarreichungsform aus mindestens einem Teil, das sowohl ein Arzneimittel als auch einen Zusatzstoff aufweist. Bei einer Tablette kann beispielsweise nur ein Teil von dem Strang abgeschnitten und dann zu einer Tablette verpresst werden. Dieses Teil wurde vorher jeweils von dem Strang gewichtsgenau abgetrennt. Die Tablette kann jedoch auch mehrere kleinere Teile aufweisen. Diese Teile wurden vorher jeweils von dem Strang gewichtsgenau abgetrennt. Bei einer mulitpartikulären Darreichungsform wird es sich immer um eine Vielzahl von Teilen handeln, die vorher gewichtgenau von dem Strang abgetrennt wurden. Diese Teile werden dann in eine Kapsel oder in einen Trinkhalm oder dergleichen eingefüllt.

Erfindungsgemäß weist jedes der Teil mindestens ein Arzneimittel und mindestens einen Zusatzstoff auf. Ein Arzneimittel im Sinne der Erfindung weist einen Wirkstoff auf. Rein beispielhaft seien hier Schmerzmittel aufgeführt. Die Zusatzstoffe können alle dem Fachmann bekannten Zusatzstoffe wie beispielsweise Füllstoffe, Bindemittel sowie Retardierungsmatrizes sein. Insbesondere handelt es sich bei dem Zusatzstoff jedoch um eine Substanz, mit der der Missbrauch des Arzneimittels verhindert werden kann.

Zusätzlich kann die Arzneimitteldarreichungsform noch Vitamine, Nahrungsmittel und/oder Nahrungsergänzungsstoffe aufweisen.

Erfindungsgemäß wird das Gemisch aus Arzneimittel und Zusatzstoffen vorzugsweise homogen gemischt und besonders bevorzugt dabei durch die Einwirkung von Scherkräften sowie Wärme in eine extrudierbare Masse verwandelt. Dieser Vorgang wird vorzugsweise in einem dem Fachmann geläufigen Extruder durchgeführt. Sodann wird das pastöse Gemisch aus Arzneimittel und Zusatzstoff aus einer Düse als Strang extrudiert.

Erfindungsgemäß wird dieser Strang nach dem Extrudieren in gewichtsgenaue Teile abgelängt, d. h. in gewichtsgenaue Teile zerteilt. Gewichtsgenau im Sinne der Erfindung bedeutet, dass das oder die Teil(e), aus dem/den letztendlich die Arzneimitteldarreichungsform hergestellt wird, die zulässigen Gewichtstoleranzen aufweist.

Die von dem Strang abzutrennende Länge, wird dazu wie folgt ermittelt. Die Querschnittsfläche des Stranges vor dem Ablängen muss bekannt sein. Insbesondere lässt sich dieser Aufgrund von Erfahrungswerten oder aufgrund von Messungen, beispielsweise des Durchmessers, ermitteln. In den seltensten Fällen entspricht der Strangquerschnitt dem Querschnitt der Düse. Mit der Querschnittsfläche und der Konzentration des Wirkstoffs in dem Strang, lässt sich die Wirkstoffmenge für eine bestimmte Längeneinheit errechnen. Die von dem Strang abzutrennende Länge ergibt sich aus der gesamten gewünschten Wirkstoffmenge geteilt durch die Wirkstoffmenge pro Längeneinheit. Wird die Querschnittsfläche gemessen, so kann dies bei der Bestimmung der Wirkstoffmenge pro Längeneinheit berücksichtigt werden.

Mit dem erfindungsgemäßen Verfahren gelingt es Darreichungsformen sicher und reproduzierbar herzustellen, die beispielsweise den Anforderungen des Deutschen Arzneimittelgesetzes, der Arzneimittel- und Wirkstoffherstellungsverordnung und/oder dem Deutschen oder Europäischen Arzneibuch genügen.

Vorzugsweise wird der Strang vor dem Ablängen gekühlt. Vorzugsweise wird der Strang soweit abgekühlt, dass er beim Schneiden nicht verschmiert; d.h. Phasen, Deformierungen, Fasern und/oder dergleichen aufweist und/oder dass beim Schneiden kein Produkt an dem jeweiligen Schneidwerkzeug hängen bleibt, was die Gewichtsgenauigkeit beeinträchtigen würde. Andererseits sollte das Produkt nicht so weit runtergekühlt werden, dass das jeweilige Schneidwerkzeug beim Schneiden Schaden nimmt bzw. zu schnell stumpf wird.
In einer besonders bevorzugten Ausführungsform weist der Strang eine hohe Steifigkeit bzw. Formstabilität des Extrudates auf, so dass der Strang sich nicht unter seinem Eigengewicht gegenüber dem extrudierten Querschnitt verformt; z. B. nicht aus einem Kreis durch Schwerkrafteinwirkung ein Oval entsteht.

In einer bevorzugten Ausführungsform wird das Schneidwerkzeug mit einer elektrischen Ladung belegt, die der Ladung des Strangmaterials entspricht. Dadurch wird das Strangmaterial von dem Schneidwerkzeug abgestoßen, so dass sich die Schneidkräfte reduzieren und Anhaftungen an dem Schneidwerkzeug vermieden werden. Dadurch wird die Gewichtsgenauigkeit der Teile und die Standzeit des Schneidwerkzeugs erhöht.

Weiterhin bevorzugt wird der Querschnitt des Strangs vor dem Ablängen kalibriert. Dadurch kann der jeweilige Querschnitt des Stranges auf ein gewünschtes Maß reduziert und/oder Schwankungen im Querschnitt des Stranges ausgeglichen werden. Der Strang weist dann eine sehr exakt, gleichbleibende Querschnittsfläche auf, was wiederum die Gewichtsgenauigkeit erhöht bzw. gegebenenfalls Querschnittsmessungen entbehrlich macht. Durch diese bevorzugte Ausführungsform ist eine besonders gewichtsgenaue Herstellung von Teilen für die jeweilige Arzneimitteldarreichungsform möglich.

Vorzugsweise wird der Strang vor dem Ablängen mit einer Abzugsvorrichtung mit einer bestimmten Geschwindigkeit abgezogen. Diese bevorzugte Ausführungsform des erfindungsgemäßen Verfahrens hat den Vorteil, dass die Geschwindigkeit mit der der Strang transportiert wird bekannt ist. Diese Geschwindigkeit muss nicht, ist jedoch vorzugsweise konstant. Vorzugsweise handelt es sich bei der Abzugsvorrichtung um eine Vorrichtung bei der maximal ein geringer Schlupf zwischen der Vorrichtung und dem abzuziehenden Strang vorkommt. Vorzugsweise handelt es sich bei der Abzugsvorrichtung um eine sogenannte Raupenabzugsvorrichtung, bei der zwei Raupen gegensinnig zu einander gedreht werden und dadurch den Strang in eine gewisse Richtung transportieren. Vorzugsweise wird der Strang dabei etwas zwischen den Raupen eingespannt. Vorzugsweise werden die Antriebsvorrichtungen von einem Antrieb angetrieben, der ein Signal absetzt mit der die Abzugsgeschwindigkeit ermittelt werden kann. Beispielsweise handelt es sich bei dem Antrieb um einen Motor mit einem Geber, beispielsweise um einen Servomotor.

Die Produktionsgeschwindigkeit des Strangs und die Geschwindigkeit der Abzugsvorrichtung sollten im Wesentlichen gleich sein, wobei vorteilhafterweise vor der Abzugsvorrichtung immer etwas Strangmaterial gepuffert ist, um zu vermeiden, dass der Strang unnötig auf Zug belastet wird und sich somit sein Querschnitt verändert. Die Pufferung kann beispielsweise durch eine einfache Schlaufe erfolgen. Dementsprechend kann die Geschwindigkeit der Abzugsvorrichtung herangezogen werden, um dass gesamte Verfahren zu regeln.

In einer besonders bevorzugten Ausführungsform wird der Strang mit einer ersten Abzugsvorrichtung vom Extruder her gezogen, dann in eine puffernde Schlaufe geführt und danach mittels einer zweiten Abzugsvorrichtung der Schneidvorrichtung zugeführt.

Besonders bevorzugt wird das Signal dies Antriebs eingesetzt, um die Schneidvorrichtung, die den Strang in gewichtsgenaue Einzelteile ablängt, zu regeln. Die Regelung erfolgt so, dass immer dann ein Schnitt erfolgt, wenn das abzuschneidende Volumen an dem Schneidwerkzeug vorbeigeführt worden ist. Bei einem konstanten Querschnitt des Stranges wird der Strang eine bestimmte Wegstrecke an der Schneidvorrichtung vorbeigeführt, bevor der Schnitt erfolgt.

Vorzugsweise handelt es sich bei dieser Schneidvorrichtung um eine rotierende Schneidvorrichtung beispielsweise um ein oder mehrere rotierende Messer, mit dem/denen der Strang taktweise abgeschnitten wird. Mögliche Messer sind eine einfache rotierende Klinge, ein Sichelmesser oder ein Kreismesser. Vorzugsweise wird das/die Messer von einem Servomotor angetrieben.

Andere mögliche Schneideinrichtungen basieren auf Strahlung.

Das Ablängen erfolgt vorzugsweise bei laufendem Vorschub des Stranges.

Vorzugsweise wird der Strang beim Ablängen geführt, diese Führung kann beispielsweise durch eine Hülse erfolgen, die den Querschnitt des Strangs zumindestens teilweise umhüllt und die für die Schneidvorrichtung als Gegenhalter wirkt. Besonders bevorzugt wird sowohl der abzulängende Teil als auch der verbleibende Strang geführt. Dadurch lässt sich ein besonders exakter Schnitt erzielen. Die Führung kann beispielsweise als zwei getrennte Hülsen ausgeführt werden, die einen etwas größeren Querschnitt aufweisen als der abzulängende Strang. Eine Hülse befindet sich in Laufrichtung des Strangs vor der Schneidvorrichtung und eines hinter der Schneidvorrichtung. Die Scheidvorrichtung taucht in den Spalt zwischen den Hülsen ein.

Vorzugsweise weist die Führung eine Temperierung auf, die es erlaubt die Temperatur des Stranges vor dem Ablängen noch einmal zu verändern.

Vorzugsweise werden die abgeschnittenen Teile nach dem Ablängen vereinzelt. Dies erfolgt insbesondere durch mechanische Energie wie beispielsweise durch einen Luftstrom, der die abgeschnitten Teile vereinzelt.

Vorzugsweise wird die ständige Produktion von Teilen überprüft. Dies kann beispielsweise durch einen Sensor am Ausgang der Schneidvorrichtung geschehen. Sobald dieser Senor keine abgeschnittenen Teile mehr detektiert, wird das Verfahren vorzugsweise unterbrochen.

Das erfindungsgemäße Verfahren eignet sich zur Herstellung jeglicher Arzneimitteldarreichungsform. Insbesondere handelt es sich doch um Arzneimittel die missbrauchgeschützt sind und die beispielsweise in der DE 103 36 400.5, DE 103 61 596.2, DE 10 2004 020 220.6, DE 10 2004 032 049.7, DE 10 2004 032 103.5, DE 10 2004 032 051.9, DE 10 2005 005 446.3, DE 10 2005 005 449.8 und DE 10 2007 011 485.2 offenbart sind. Diese Patentanmeldungen werden hiermit als Referenz eingeführt und gelten somit als Teil der Offenbarung.

Ein weiterer Gegenstand der vorliegenden Erfindung ist eine Vorrichtung zur gewichtsgenauen Ablängung eines Stranges, der aus einer Düse extrudiert wird, mit einem Mittel zur Ablängung des Stranges, die ein Mittel zur Kalibrierung des Querschnitts des Stranges aufweist.

Die gesamte zu dem erfindungsgemäßen Verfahren gemachte Offenbarung gilt für die erfindungsgemäße Vorrichtung gleichermaßen.

Mit der erfindungsgemäßen Vorrichtung ist es möglich, einen Strang in gewichtsgenaue Teile abzulängen.

Gewichtsgenau im Sinne der Erfindung bedeutet, dass das Istgewicht der abgetrennten Teile dem Deutschen Arzneimittelgesetz, der Arzneimittel- und Wirkstoffherstellungsverordnung und/oder dem Deutschen oder Europäischen Arzneibuch genügen.

Erfindungsgemäß weist die Vorrichtung eine Kalibrierung auf, mit der der Querschnitt des Strangs auf einen gewünschten Sollwert geformt wird. Vorzugsweise ist vor der Kablibrierung eine Kühlung angeordnet, die den Strang soweit abkühlt, dass er nach der Kalibrierung in der kalibrierten Form bleibt und sich nicht mehr oder nicht nennenswert verändert

Vorzugsweise weist die erfindungsgemäße Vorrichtung ein Mittel zum Abzug des Stranges auf. Mit diesem Mittel ist es möglich, den Strang mit einer bestimmten Geschwindigkeit, die nicht konstant sein muss, jedoch vorzugsweise konstant ist, zu transportieren. Vorzugsweise weist dieses Antriebsmittel einen Antrieb auf, der ein Signal absetzt, mit dem die Abzugsgeschwindigkeit ermittelbar ist. Vorzugsweise handelt es sich bei dem Mittel um sogenannte Abzugsraupen, die gegenüberliegend am Querschnitt des Stranges angeordnet werden. Diese einstellbaren Raupen weisen kein oder nur ein sehr geringen Schlupf gegenüber dem Strang auf, so dass die Drehgeschwindigkeit der Walzen der Transportgeschwindigkeit des Stranges entspricht. Vorzugsweise sind die Raupen gegen den Strang vorspannbar und/oder an unterschiedliche Querschnitte anpassbar ist. Vorzugsweise weist das Transportmittel einen Antrieb auf, der eine Signal absetzt, mit dem diese Abzugsgeschwindigkeit ermittelbar ist.

Vorzugsweise weist die Vorrichtung eine erste Abzugseinrichtung zum Abziehen des Stranges vom Extruder, eine puffernde Schlaufe und eine zweite Abzugseinrichtung zur Zuführung zur Schneidvorrichtung auf.

Vorzugsweise weist die erfindungsgemäße Vorrichtung weiterhin eine Steuerungseinheit auf, die das von dem Antrieb abgesetzte Signal empfängt und vorzugsweise mit diesem Signal die Schneidvorrichtung, mit der der Strang abgelängt wird, steuert. Weiterhin bevorzugt wird auch die Geschwindigkeit, mit der der Strang produziert wird, mit der Abzugsgeschwindigkeit geregelt.

Vorzugsweise handelt es sich bei dieser Schneidvorrichtung um ein rotierendes Messer. Bei dieser bevorzugten Ausführungsform wird die Rotationsgeschwindigkeit des Messers von der Vorrichtung geregelt.

Weiterhin bevorzugt wird der Strang beim Schneiden geführt. Diese Führung erfolgt beispielsweise in einer Führungshülse, die den Strang zumindestens teilweise umschließt.

Vorzugsweise wird sowohl der abzuschneidende Teil als auch der verbleibende Strang geführt, dadurch ist ein besonders exakter Schnitt möglich.

In einer weiteren bevorzugten Ausführungsform ist der Strang nach dem Abzug und vor dem Ablängen temperierbar, um eine optimale Temperatur des Stranges beim Ablängen einstellen zu können.

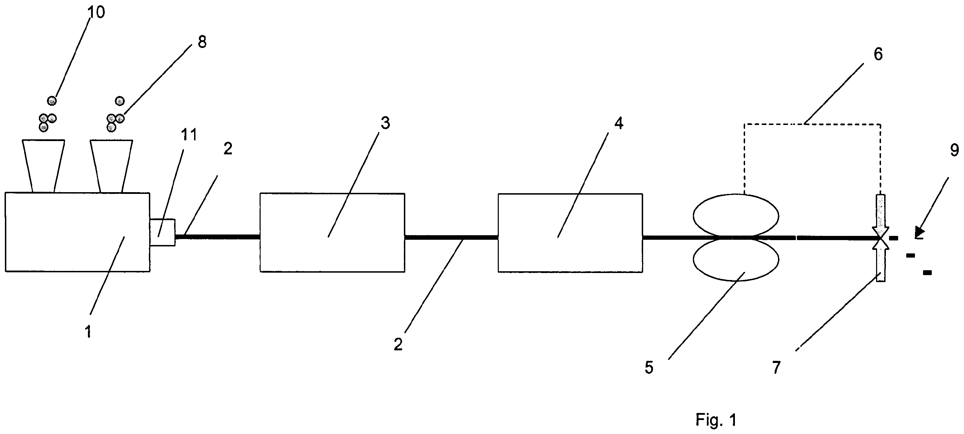

Im Folgenden wird die Erfindung anhand der einzigen Figur 1 erläutert. Diese Erläuterungen sind lediglich beispielhaft und schränken den allgemeinen Erfindungsgedanken nicht ein. Diese Erläuterungen gelten sowohl für das erfindungsgemäße Verfahren als auch die erfindungsgemäße Vorrichtung gleichermaßen.

Figur 1 zeigt eine mögliche Ausführungsform des erfindungsgemäßen Verfahrens bzw. der erfindungsgemäßen Vorrichtung. Der Extruder 1 wird kontinuierlich mit einem Arzneimittel 8 und einem Zusatzstoff 10 befüllt. Diese können separat oder in Mischung vorliegen und müssen dem Extruder auch nicht an derselben Stelle zugegeben werden. In dem Extruder werden das Arzneimittel und der Zusatzstoff unter Einwirkung von Wärme und Scherkräften zu einer homogenen pastösen Masse verarbeitet. Diese Masse wird mit der Düse 11 kontinuierlich zu einem Strang 2 extrudiert. Die Querschnittsform des Strangs richtet sich beispielsweise nach der Form der später herzustellenden Tablette. Dieser Strang 2 wird zunächst in einem Kühler 3 von der Extrusions- auf eine niedrigere Temperatur abgekühlt, die ca. 55-45 % der Extrusionstemperatur beträgt. Zwischen der Extruderdüse 11 und dem Kühler 2 weist der Strang 2 vorzugsweise eine in ihrer Größe einstellbare Schlaufe (nicht dargestellt) als Puffer auf. Innerhalb des Kühlers befindet sich ein beispielsweise Endlosförderband, das den Strang 2 durch den Kühler fördert. Auch auf diesem Förderband kann der Strang in Schlaufen liegen, um die Länge des Kühlers zu reduzieren und/oder die Durchlaufzeit zu ändern. Nach dem Kühlen wird der Strang 2 vorzugsweise kalibriert. Dabei wird der Strangquerschnitt auf einen im Wesentlichen über die Länge des Stranges gleichmäßigen Querschnitt reduziert. Vorzugsweise weist die Kalibrierung mindestens ein Rollenpaar auf, das in einem gewissen Abstand zueinander angeordnet ist. Der Strang wird durch das jeweilige Rollenpaar geführt und dabei in seinem Querschnitt reduziert. Die Rollen sind synchron angetrieben und gegebenenfalls temperierbar. Nach der Kalibrierung wird der Querschnitt des Stranges vorzugsweise vermessen. Die Vermessung erfolgt mit mehreren Lasern. Das Resultat dieser Messung kann zur Steuerung der Schneidvorrichtung 7 herangezogen werden. Stromabwärts von der Kalibrierung ist eine Abzugsvorrichtung 5 in Form von zwei in ihrem Abstand einstellbaren Raupen angeordnet, wobei sich die untere Raupe im und die obere Raupe gegen den Uhrzeigersinn dreht. Der Antrieb der Raupen erfolgt durch einen Servomotor, der ein Signal absetzt, mit dem die Drehgeschwindigkeit und damit die Abzugsgeschwindigkeit des Strangs ermittelbar ist. Die Geschwindigkeit der Raupen ist einstellbar und muss nicht konstant sein. Die Raupen werden leicht gegen den Strang vorgespannt, um Schlupf möglicht zu vermeiden. Das Signal des Antriebs der Raupen wird, wie durch die gestrichelte Linie dargestellt, an eine Steuerung übergeben, die die Schneidvorrichtung steuert. Aufgrund der Konzentration (mg/ Volumen) des Wirkstoffs in dem Strang und der Kenntnis der Querschnittsfläche nach dem Kalibrieren, errechnet die Steuerung die Länge des jeweils abzulängenden Strangs, um eine Arzneimitteldarreichtungsform oder einen Teil einer gewissen Arzneimitteldarreichungsform mit einer bestimmten Menge an Wirkstoff zu erhalten. Aufgrund der Transportgeschwindigkeit kann demnach der Zeitpunkt errechnet werden an dem die Abtrennung des jeweiligen Teiles erfolgen muß. Die Abtrennung erfolgt mit einer Trennvorrichtung, in dem vorliegenden Fall mit einem rotierend angetriebenen Messerhalter, an dem eine, zwei oder mehrere Klingen angeordnet sind. Die Steuerung steuert nun die Drehzahl des Messerhalters so, dass gewichtsgenaue Teile abgelängt werden. Die Steuerung berücksichtigt dabei in jedem Fall die Transportgeschwindigkeit des Strangs gegebenenfalls auch das Resultat der Durchmessermessung vor der Abzugsvorrichtung, insbesondere dann, wenn der Querschnitt des Strangs über dessen Länge stark schwankt. Während des Schneidens wird der Strang in einer Hülse geführt, deren Querschnitt etwas größer ist als der Querschnitt des Strangs. Die Hülse führt den Strang stromaufwärts und stromabwärts der Schneidvorrichtung. Diese Hülse wird mit einer Flüssigkeit temperiert, um den Strang gegebenenfalls vor dem Schneiden noch einmal Erwärmen oder Abkühlen zu können. Dafür ist vor der Hülse eine Temperaturmessung angeordnet, die die Oberflächentemperatur des Strangs berührungslos mißt. Die Temperatur des Stranges sollte so gewählt werden, dass eine möglichst lange Standzeit der Messer und jeweils ein sauberer Schnitt ohne Rückstände und Verformungen gewährleistet ist. Vorzugsweise erfolgt die Ablängung mittels der Trennvorrichtung demnach nicht nur durch Schneiden, sondern auch durch Abschlagen. Nach der Schneidvorrichtung ist noch eine Röhrenführung angeordnet, die die abgeschnittenen Teile vereinzelt. Ein in diesem oder vor diesem Bereich angeordneter Sensor detektiert permanent, ob Teile 9 abgelängt werden und hält das Herstellungsverfahren an, sollte dies nicht der Fall sein. Nach dem Ablängen werden die gewichtsgenau hergestellten Teile 9 in einer Puffereinrichtung aufgefangen und gelangen dann, wenn eine Umformung gewünscht ist, in eine Presse, in der sie ihre abschließende Form erhalten. Es ist aber auch möglich, dass mehrere Teile zu einer Arzneimitteldarreichungsform verarbeitet werden. In diesem Fall wird die gewünschte Anzahl zusammengefasst und dann weiterverarbeitet. Die Zusammenfassung kann rein numerisch erfolgen, da die Teile gewichtsgenau sind und dadurch eine gewisse Anzahl an Teilen auch die gewünschte Arzneimittelmenge ergibt. So können Tabletten, die aus mehreren Teilen verpresst werden oder multipartikuläre Tabletten hergestellt werden.

### Bezugszeichenliste:

- 1: Extruder
- 2: Strang
- 3: Kühlung
- 4: Kalibrierung
- 5: Abzugsvorrichtung, Raupenabzug
- 6: Signal
- 7: Schneidvorrichtung
- 8: Arzneimittel
- 9: Abglängte Teile
- 10: Zusatzstoffe
- 11: Düse

## Patentansprüche

1. Verfahren zur Herstellung einer Arzneimitteldarreichungsform, die aus mindestens einem Teil (9) besteht, das jeweils mindestens ein Arzneimittel (8) und mindestens einen Zusatzstoff (10) aufweist, wobei das Arzneimittel (8) und der Zusatzstoff (10) gemischt und aus einer Düse (11) als Strang (2) extrudiert wird, **dadurch gekennzeichnet, dass** der Strang (2) in gewichtsgenaue Teile (9) abgelängt wird und dass aus ihnen unmittelbar, ohne weitere Schritte zur Einhaltung eines Sollgewichtes, eine Arzneimitteldarreichungsform hergestellt wird.

2. Verfahren nach Anspruch 1, **dadurch gekennzeichnet, dass** der Strang (2) vor dem Ablängen gekühlt wird.

3. Verfahren nach einem der voranstehenden Ansprüche, **dadurch gekennzeichnet, dass** der Querschnitt des Strangs (2) vor dem Ablängen kalibriert wird, wobei das Kalibrieren (4) vorzugsweise nach dem Kühlen (3) erfolgt.

4. Verfahren nach einem der voranstehenden Ansprüche, **dadurch gekennzeichnet, dass** der Strang vor dem Ablängen mit einer Abzugsvorrichtung (5) mit einer bestimmten Geschwindigkeit abgezogen wird und der Strang vor dem Abziehen vorzugsweise gepuffert wird.

5. Verfahren nach Anspruch 3 oder 4, **dadurch gekennzeichnet, dass** von der Abzugsvorrichtung ein Signal abgesetzt wird, mit dem die Abzugsgeschwindigkeit ermittelt wird und dass diese Abzugsgeschwindigkeit zur Steuerung der Schneidvorrichtung (7), mit der der Strang abgelängt wird, herangezogen wird.

6. Verfahren nach einem der voranstehenden Ansprüche, **dadurch gekennzeichnet, dass** die Ablängung durch eine rotierende Schneidvorrichtung erfolgt, wobei die Transportgeschwindigkeit vorzugsweise die Drehzahl der Schneidvorrichtung regelt.

7. Verfahren nach einem der voranstehenden Ansprüche, **dadurch gekennzeichnet, dass** der Strang beim Ablängen geführt wird und vorzugsweise der Durchmesser des Strangs vor dem Ablängen überprüft wird.

8. Verfahren nach einem der voranstehenden Ansprüche, **dadurch gekennzeichnet, dass** der Strang nach dem Abziehen und vor dem Ablängen noch einmal temperiert wird.

9. Vorrichtung zur gewichtsgenauen Ablängung eines Stranges (2), der aus einer Düse (11) extrudiert ist, mit einem Mittel (7) zur Ablängung des Stranges, **dadurch gekennzeichnet, dass** sie ein Mittel (4) zur Kalibrierung des Querschnitts des Stranges (2) aufweist.

10. Vorrichtung nach Anspruch 9, **dadurch gekennzeichnet, dass** bezogen auf die Transportrichtung des Strangs vor der Kalbrierung (4) eine Kühlung (3) angeordnet ist und sie vorzugsweise ein Mittel (5) zum Abzug des Stranges (2) aufweist.

11. Vorrichtung nach einem der voranstehenden Ansprüche, **dadurch gekennzeichnet, dass** es ein weiteres Mittel zum Abzug des Stranges (2) aufweist, wobei der Strang zwischen den Abzugsmitteln vorzugsweise in Form einer Schleife gepuffert ist.

12. Vorrichtung nach Anspruch 10 oder 11, **dadurch gekennzeichnet, dass** das Mittel (5) einen Antrieb aufweist, der ein Signal absetzt, mit dem die Abzugsgeschwindigkeit ermittelbar ist und dass sie vorzugsweise eine Steuerungseinheit aufweist, die aufgrund des Signals das Mittel (7) steuert.

13. Vorrichtung nach einem der voranstehenden Ansprüche, **dadurch gekennzeichnet, dass** das Mittel (7) ein rotierendes Messer ist, wobei die Drehzahl des Messers vorzugsweise geregelt ist.

14. Vorrichtung nach einem der voranstehenden Ansprüche, **dadurch gekennzeichnet, dass** der Strang (2) beim Schneiden geführt ist.

15. Vorrichtung nach einem der voranstehenden Ansprüche, **dadurch gekennzeichnet, dass** der Strang (2) nach dem Abzug und vorm und/oder beim Ablängen temperierbar ist.

## Claims

1. Method for producing a medicament administration form which consists of at least one part (9), each part containing at least one medicament (8) and at least one additive (10), wherein the medicament (8) and the additive (10) are mixed and extruded from a die (11) as a strand (2), **characterised in that** the strand (2) is cut to length into parts of precise weight (9) and that a medicament administration form is produced therefrom directly, without any further steps to ensure conformance to a target weight.

2. Method according to claim 1, **characterised in that** the strand (2) is cooled before being cut to length.

3. Method according to either of the preceding claims, **characterised in that** the cross section of the strand (2) is calibrated before being cut to length, wherein the calibration (4) preferably takes place before the cooling (3).

4. Method according to any one of the preceding claims, **characterised in that** the strand is drawn off at a certain speed by a drawing device (5) before being cut to length, and the strand is preferably buffered for being drawn off.

5. Method according to claim 3 or 4, **characterised in that** signal is output by the drawing device, which signal is used to determine the drawing off speed, and that this drawing off speed is used for controlling the cutting device (7) with which the strand is cut to length.

6. Method according to any one of the preceding claims, **characterised in that** the cutting to length is carried out by a rotating cutting device, wherein the transport speed preferably regulates the rotating speed of the cutting device.

7. Method according to any one of the preceding claims, **characterised in that** the strand is guided during cutting to length and the diameter of the strand is checked preferably before the cutting to length.

8. Method according to any one of the preceding claims, **characterised in that** the temperature of the strand is regulated after it is drawn off and again before it is cut to length.

9. Device for cutting a strand (2) to a length with precise weight, which strand is extruded from a die (11), said device comprising a means (7) for cutting the strand to the length, **characterised in that** it comprising a means (4) for calibrating the cross section of the strand (2).

10. Device according to claim 9, **characterised in that** a cooling arrangement (3) is arranged upstream of the calibration (4) with respect to the feed direction of the strand, and said cooling arrangement preferably has a means (5) for drawing out the strand (2).

11. Device according to any one of the preceding claims, **characterised in that** it has a second means for drawing out the strand (2), wherein the strand is buffered preferably in the form of a loop between the first and the second drawing out means.

12. Device according to claim 10 or 11, **characterised in that** the means (5) has a drive unit that outputs a signal with which the drawing out speed can be determined, and that it preferably has a control unit which controls the means (7) on the basis of the signal.

13. Device according to any one of the preceding claims, **characterised in that** the means (7) is a rotating knife, wherein the speed of the knife is preferably regulated.

14. Device according to any one of the preceding claims, **characterised in that** the strand (2) is guided during cutting.

15. Device according to any one of the preceding claims, **characterised in that** the temperature of the strand (2) can be regulated after it has been drawn out and

## Revendications

1. Procédé de production d'une forme pharmaceutique d'un médicament qui se compose d'au moins une partie (9), qui possède respectivement au moins un médicament (8) et au moins un additif (10), le médicament (8) et l'additif (10) étant mélangés et extrudés depuis une buse (11) sous la forme d'un cordon (2), **caractérisé en ce que** le cordon (2) est tronçonné en parties (9) au poids précis et **en ce qu'**une forme pharmaceutique du médicament est produite directement à partir de celles-ci, sans étapes supplémentaires destinées au respect d'un poids voulu.

2. Procédé selon la revendication 1, **caractérisé en ce que** le cordon (2) est refroidi avant le tronçonnage.

3. Procédé selon l'une des revendications précédentes, **caractérisé en ce que** la section transversale du cordon (2) est calibrée avant le tronçonnage, le calibrage (4) s'effectuant de préférence après le refroidissement (3).

4. Procédé selon l'une des revendications précédentes, **caractérisé en ce que** le cordon est extrait avant le tronçonnage à l'aide d'un dispositif d'extraction (5) avec une vitesse donnée et le cordon est de préférence mis en tampon avant l'extraction.

5. Procédé selon la revendication 3 ou 4, **caractérisé en ce que** le dispositif d'extraction délivre un signal à l'aide duquel est déterminée la vitesse d'extraction, et **en ce que** cette vitesse d'extraction est utilisée pour commander le dispositif de coupe (7) avec lequel est tronçonné le cordon.

6. Procédé selon l'une des revendications précédentes, **caractérisé en ce que** le tronçonnage est réalisé par un dispositif de coupe rotatif, la vitesse de rotation du dispositif de coupe étant de préférence régulée par la vitesse de transport.

7. Procédé selon l'une des revendications précédentes, **caractérisé en ce que** le cordon est guidé lors du tronçonnage et le diamètre du cordon est de préférence contrôlé avant le tronçonnage.

8. Procédé selon l'une des revendications précédentes, **caractérisé en ce que** le cordon est encore une fois équilibré en température après l'extraction et avant le tronçonnage.

9. Dispositif de tronçonnage au poids précis d'un cordon (2) qui est extrudé depuis une buse (11), comprenant des moyens (7) servant à tronçonner le cordon, **caractérisé en ce qu'**il possède des moyens (4) servant à calibrer la section transversale du cordon (2).

10. Dispositif selon la revendication 9, **caractérisé en ce qu'**un système de refroidissement (3) est installé avant le calibrage (4) en référence au sens de transport du cordon, et il possède de préférence des moyens (5) servant à l'extraction du cordon (2).

11. Dispositif selon l'une des revendications précédentes, **caractérisé en ce qu'**il possède des moyens supplémentaires pour l'extraction du cordon (2), le cordon étant mis en tampon entre les moyens d'extraction de préférence sous la forme d'une boucle.

12. Dispositif selon la revendication 10 ou 11, **caractérisé en ce que** les moyens (5) possèdent un mécanisme d'entraînement qui délivre un signal à l'aide duquel peut être déterminée la vitesse d'extraction, et **en ce qu'**il possède de préférence une unité de commande qui commande les moyens (7) en fonction du signal.

13. Dispositif selon l'une des revendications précédentes, **caractérisé en ce que** les moyens (7) sont une lame tournante, la vitesse de rotation de la lame étant de préférence régulée.

14. Dispositif selon l'une des revendications précédentes, **caractérisé en ce que** le cordon (2) est guidé lors de la coupe.

15. Dispositif selon l'une des revendications précédentes, **caractérisé en ce que** le cordon (2) peut faire l'objet d'un équilibrage thermique après l'extraction et avant le et/ou lors du tronçonnage.
